# EUROPÄISCHE PATENTANMELDUNG

(11) **EP 3 143 971 A1**
(43) Veröffentlichungstag der Anmeldung: **22.03.2017**
(21) Anmeldenummer: 16188402.8
(22) Anmeldetag: 12.09.2016
(51) Int. Cl.: A61F 5/01, A61F 5/02, A61F 5/03

(54) **ORTHESE ZUR BECKENSTABILISATION**

(30) Priorität: 18.09.2015 CH 13542015
(71) Anmelder: Ortho-Team AG, 3008 Bern (CH); Universität Bern, 3012 Bern (CH)
(72) Erfinder: Bosshard, Adrian, 3065 Bolligen (CH); Widmer, Sandra, 4656 Starrkirch-Wil (CH); Kohl, Sandro, 3007 Bern (CH); Keel, Marius Johann Baptist, 8002 Zürich (CH); Kohlhof, Hendrik, 50678 Köln (DE)
(74) Vertreter: BOVARD AG

(57) **Zusammenfassung**

Eine Orthese zur Beckenstabilisation weist eine Gurteinheit, die zum Umschliessen des Beckens vorgesehen ist, und eine Zugeinrichtung, die zum Straffen der Gurteinheit vorgesehen ist, auf. Die Gurteinheit umfasst ein oberes Gurtband (1), das einen oberen Beckenbereich umschliesst, und ein unteres Gurtband (2), das einen unteren Beckenbereich umschliesst. Die Zugeinrichtung umfasst eine vordere Zugeinheit mit einer Befestigungseinrichtung, wobei die vordere Zugeinheit an einem vorderen Beckenbereich der Gurteinheit angeordnet ist und das obere und das untere Gurtband in einem vorderen Beckenbereich durch Zug strafft. Dabei verbindet die Befestigungseinrichtung die gegenüberliegende Enden (3, 4; 5, 6) des oberen und des unteren Gurtbands im vorderen Beckenbereich miteinander und befestigt diese unter Zug. Die Zugeinrichtung weist weiter eine hintere Zugeinheit (8, 9), die in einem mittleren Bereich (7) der Bänder am oberen Gurtband (1) und am unteren Gurtband (2) angeordnet ist und zum Straffen eines hinteren Beckenbereichs der Gurteinheit vorgesehen ist, auf. Die hintere Zugeinheit wird durch eine Befestigungseinheit unter Zug befestigt.

## Beschreibung

Die vorliegende Erfindung betrifft eine Orthese zur Beckenstabilisation, die eine Gurteinheit um einen Beckenbereich und eine Zugeinrichtung zum Straffen der Gurteinheit umfasst. Eine solche Orthese kommt z. B. zur Stabilisierung von Beckenfrakturen zum Einsatz.

Verletzungen des Beckens bilden ca. 3% der unfallbedingten Skelett-Verletzungen und bedürfen einer frühen Erkennung während der Erstversorgung am Unfallort, da eine Instabilität des Beckens grossen Einfluss auf die Sterblichkeit des Betroffenen als Folge der Traumatisierung hat. Bei stark verletzten Traumapatienten kann ein hämorrhagischer Schock, der z. B. durch Reissen des sakralen Venenplexus, Blutung aus der Bruchstelle oder Arterienverletzungen hervorgerufen wird, schnell zum Tod führen. Derartig verursachte Blutungen können durch eine rechtzeitige Stabilisierung des Beckens unter Kontrolle gebracht werden. Dabei wird eine Schlinge oder ein Gurt verwendet, der so früh als möglich um das Becken anzulegen ist. Es hat sich gezeigt, dass die dadurch erreichte Tamponierwirkung nicht nur die Blutung verringert, sondern auch die benötigten Transfusionen reduziert und Klinikaufenthalte verkürzt. Auch kann die Beckenstabilisation mit einem Gurt genügen, um eine ausreichende hämodynamische Stabilität während mehrerer Stunden oder sogar Tagen zu erreichen bis eine definitive Behandlung erfolgen kann.

Bei einer Stabilisation des Beckens muss die Anatomie des Beckenrings beachtet werden, die durch das hinten liegende Kreuzbein, zwei symmetrisch daran mittels Bändern anschliessende flügelartige Darmbeine und die vorne liegenden Schambeine mit der Schambeinfuge gegeben ist. Die robuste Struktur des Beckens ermöglicht die Übertragung von axialen Kräften vom Oberkörper zu den unteren Extremitäten, während rotierende und vertikale Kräfte auf das Becken einwirken. Der vorne liegende Schambeinbereich ist anfällig für Verletzungen, die zu einer Öffnung des vorderen Bereichs des Beckenrings führen können. Verletzungen im hinten liegende Bereich um das Kreuzbein beeinträchtigen u. a. die hämodynamische Stabilität und das Nervensystem.

Die meisten zur Beckenstabilisation verwendeten Gurte beruhen darauf, das Becken von der Vorderseite her zu komprimieren. Dabei wird ein Gurt unter das Gesäss des liegenden Patienten gebracht und festgezogen, um einen auf der Vorderseite angreifenden Zugmechanismus zu aktivieren.

Aus der EP 1337207 B1 ist z. B. ein Orthesegurt bekannt, der bei Beckenbrüchen an einem Unfallort, beim Transport eines Unfallopfers und auch in der Notaufnahme von Krankenhäusern Verwendung finden kann. Der Orthesegurt besteht aus einem länglichen breiten Gurtelement, dessen Enden in üblicher Weise um den Patienten herum aneinander befestigt werden. Darüber hinaus werden die Enden über eine Spannvorrichtung miteinander verbunden, mit der der Gurt um das Becken des Patienten gestrafft werden kann. Die Spannvorrichtung wird durch einen Seilzug gebildet, bei dem Seile über einen Rollensatz mit mehreren Rollen mehrfach zwischen den Enden des Gurtelements hin und her geführt und am Ende von einem Griff gehalten werden. Beim Ziehen am Griff verteilt sich die Zugkraft auf das zwischen den Gurtenden verlaufende Seil und zieht die Enden gleichmässig auf einander zu. Die Breite des Gurtelements überdeckt dabei einen breiten Bereich des Beckens, so dass dieser unzugänglich ist für eine Behandlung des Patienten. Ferner wird der Kompressionsdruck undifferenziert über die gesamte Breite des Gurtelements auf das Becken übertragen und eine Feineinstellung für obere und untere Beckenbereiche ist nicht möglich.

Schwere Verletzungen umfassen häufig auch Verletzungen des hinteren Kreuzbeinbereichs. Eine im vorderen Bereich ausgelöste Kompression eines Beckengurts kann dabei zu einer unerwünschten Dehnung des hinteren Beckenbereichs führen. Eine Instabilität des hinteren Beckenbereichs kann jedoch leicht zu einem hämorrhagischen Schock führen, da dort der Venenplexus angeordnet ist.

Aus der WO 2014/028248 ist ein Beckengurt zur Stabilisierung bei Beckenfrakturen bekannt, der zwei unabhängige parallel verlaufende Gurtbänder umfasst, die getrennt voneinander festgezogen werden. Ein oberes Band soll zur Kompression im Bereich des Kreuzbeins und des vorderen Abdomens dienen und wird hierfür auf Höhe des Kreuzbeins vorgesehen. Das obere Band weist ein hinteres Kompressionskissen auf, das auf das Weichgewebe des Kreuzbeins drückt. Weiter kann es ein vorderes Kompressionskissen aufweisen, das auf die Verzweigung der Bauchaorta und die Beckenarterien wirken soll, um den Blutfluss zu entfernter gelegenen Arterienbereichen zu reduzieren. Ein unteres Band dient zur Stabilisierung der Fraktur und wird hierfür auf Höhe des Oberschenkelkopfes platziert. Die beiden Bänder werden individuell festgezogen, so dass das untere Band für eine optimal Stabilisierung des Beckens und das obere Band für einen optimalen Druck der Kompressionskissen auf das hinten liegende Kreuzbein eingestellt werden kann. Darüber hinaus, bleibt ein Zwischenraum zwischen den Bändern frei zugänglich. Allerdings erfolgt das Festziehen der Bänder ebenfalls von der vorderen Seite, wodurch die oben beschriebene Gefahr einer unerwünschten Dehnung des hinteren Beckenbereichs bestehen bleibt. Zudem erfolgt die Befestigung der Bänder in der angezogenen Stellung allein über einen Klettverschluss, so dass nur eine sehr grobe Einstellung der auf die Bänder wirkenden Zugkraft erfolgen kann. Die zur Blutstillung erforderlichen Kompressionskissen überdecken im hinteren Bereich einen Grossteil des Beckens.

Es ist eine Aufgabe der vorliegenden Erfindung eine Orthese zur Beckenstabilisation bereitzustellen, die eine optimale Stabilisierung oder Fixierung unterschiedlicher Beckenbereiche und eine zuverlässige Blutstillung ermöglicht, die einfach und schnell um das Becken eines Patienten angelegt werden kann, die eine Kompression ohne grossen Kraftaufwand erlaubt sowie die weitere Versorgung und Behandlung des Patienten nicht behindert.

Diese Aufgabe wird von der Erfindung durch eine Orthese zur Beckenstabilisation nach Anspruch 1 gelöst. Vorteilhafte Ausgestaltungen und verschiedene Ausführungsbeispiele sind in den abhängigen Ansprüchen beschrieben.

Eine Orthese zur Beckenstabilisation nach der vorliegenden Erfindung weist eine Gurteinheit, die zum Umschliessen des Beckens vorgesehen ist, und eine Zugeinrichtung, die zum Straffen der Gurteinheit vorgesehen ist, auf. Die Gurteinheit umfasst ein oberes und ein unteres Gurtband, die jeweils um das Becken geschlungen werden. Das obere Gurtband umschliesst einen oberen Beckenbereich und das unteres Gurtband einen unteren Beckenbereich. Oben und unten soll in diesem Zusammenhang auf einen Patienten bezogen verstanden werden, so dass ein oberes Gurtband dem Kopf näher ist und vorzugsweise im Bereich des Kreuzbeins um das Becken zu liegen kommt und das untere Gurtband den Beinen näher ist und vorzugsweise im Bereich der Oberschenkelköpfe zu liegen kommt.

Die Zugeinrichtung weist eine vordere Zugeinheit mit einer Befestigungseinrichtung zum Fixieren der Zugeinheit unter Zug auf. Die vordere Zugeinheit ist an einem vorderen Beckenbereich der Gurteinheit angeordnet. Das heisst, in einem einen Patienten umschliessenden Zustand kommt die vordere Zugeinheit am vorderen Beckenbereich des Patienten zu liegen, also z. B. über dem Schambeinbereich. Die vordere Zugeinheit strafft das obere und das untere Gurtband in einem vorderen Beckenbereich durch Zug an der Zugeinheit. Im wesentliche greift die vordere Zugeinheit an Endbereichen der Gurtbänder an und reduziert durch Zug, z. B. an einem der Endbereiche, den Durchmesser der Gurtbänder und damit den Durchmesser der Gurteinheit. Bevorzugt umfasst die vordere Zugeinheit für das obere und das untere Gurtband getrennte vordere Zugmittel, so dass das obere und das untere Gurtband getrennt gestrafft werden können. Vorteilhaft ist das untere Gurtband breiter als das obere Gurtband. Vorzugsweise ist das untere Band eineinhalb- bis zweimal breiter als das obere Band.

Alternativ ist es auch möglich ein gemeinsames vorderes Zugmittel vorzusehen, das gleichzeitig einen Zug auf das obere und das untere Gurtband aufbringt.

Die Befestigungseinrichtung verbindet die gegenüberliegenden Enden respektive Endbereiche des oberen Gurtbands und die Enden respektive Endbereiche des unteren Gurtbands im vorderen Beckenbereich miteinander und befestigt diese unter Zug. Als ein Beispiel für eine Zugeinheit mit Befestigungseinrichtung kann an einem Endbereich der Gurtbänder eine Durchführung, z. B. ein Schlitz oder Ring, vorgesehen sein, durch die der gegenüberliegende Endbereich hindurchgeführt und angezogen wird. Zur Befestigung kann z. B. ein Verschluss in Form eines Klettverschlusses mit Widerhaken und Schlaufen vorgesehen sein, der in bekannter Weise durch aufeinander legen von Widerhaken- und Schlaufenbereichen fixiert wird.

Bei der Verwendung einer Orthese nach der Erfindung wird wie üblich die Gurteinheit in einem geöffneten Zustand der Gurtbänder im Beckenbereich um einen Patienten oder ein Unfallopfer gelegt. Mit der Befestigungseinrichtung werden jeweils die sich gegenüberliegenden Endbereiche der Gurtbänder verbunden, so dass das obere Gurtband und das untere Gurtband das Becken umschliessen. Mit der vorderen Zugeinheit werden die Bänder gestrafft und durch die Befestigungseinrichtung in der gestrafften Position fixiert.

Nach der vorliegenden Erfindung weist die Zugeinrichtung zusätzlich zur vorderen Zugeinheit eine hintere Zugeinheit und eine Befestigungseinheit für die hintere Zugeinheit auf. Die hintere Zugeinheit ist in einem mittleren Bereich der Bänder am oberen und am unteren Gurtband angeordnet. Die hintere Zugeinheit liegt somit zwischen den Endbereichen der Bänder, an welchen die vordere Zugeinheit angreift. Ist die Orthese um einen Patienten gelegt, kommt die hintere Zugeinheit auf der anderen Seite des Patienten als die vordere Zugeinheit zu liegen und befindet sich somit an einem hinteren Beckenbereich. Die hintere Zugeinheit strafft die Gurtbänder der Gurteinheit während sie an einem hinteren Beckenbereich angeordnet ist und wirkt somit auf den hinteren Beckenbereich, wobei sich der Durchmesser der Gurteinheit weiter verringert und den hinteren Beckenbereich komprimiert. Mit der Befestigungseinheit wird die hintere Zugeinheit unter Zug befestigt. Hierfür kann z. B. ebenfalls ein Klettverschluss verwendet werden.

Bei der Orthese nach der vorliegenden Erfindung wird eine erste Schliesskraft zur Stabilisierung des Beckens beim Anlegen der Orthese durch die vordere Zugeinheit im vorderen Bereich aufgebracht. Durch die hintere Zugeinheit ist es bei der Orthese möglich, eine verengende Schliesskraft auch auf den hinteren Beckenbereich auszuüben und dadurch eine Stabilisierung zu erzielen, welche Blutungen im hinteren Beckenbereich eindämmt oder sogar unterbinden kann. Das Risiko eines hämorrhagischer Schocks ist dadurch deutlich geringer als bei herkömmlichen Orthesen. Dabei ist es besonders vorteilhaft, dass die verengende Kraft auf das obere und das untere Gurtband wirkt aber zugleich ein Zwischenraum zwischen den Bändern verbleibt, so dass der hintere Beckenbereich für eine weitere Untersuchung oder Behandlung zugänglich bleibt ohne, dass die Orthese abgenommen werden muss. Die einzelnen Bänder der Orthese wirken an gezielt ausgewählten Bereichen auf das Becken, um eine optimale Stabilisierung des gesamten Beckenbereichs zu ermöglichen. Bereiche, die für die Stabilisierung weniger relevante sind, können frei bleiben.

Bei einer vorteilhaften Ausgestaltung der Orthese nach der Erfindung ist die hintere Zugeinheit übergreifend am oberen und unteren Gurtband angeordnet, so dass die hintere Zugeinheit zum Straffen der mittleren Bandbereiche gleichzeitig auf das obere und das untere Gurtband wirkt. Dadurch kann die hintere Zugeinheit durch einen einzigen Handgriff festgezogen werden, wodurch ein Patient schneller für einen Transport bereit wird.

In einer Ausführungsform der Orthese nach der Erfindung ist es vorgesehen, dass die vordere Zugeinheit ein erstes vorderes Zugmittel am oberen Gurtband für einen vorderen Beckenbereich und ein zweites vorderes Zugmittel am unteren Gurtband ebenfalls für einen vorderen Beckenbereich aufweist. Dabei können das erste und das zweite Zugmittel jeweils eine eigene Befestigungseinheit umfassen, die jeweils gegenüberliegende Enden des oberen beziehungsweise des untere Gurtbands im vorderen Beckenbereich miteinander verbindet und unter Zug fixiert. Somit kann in einem ersten Schritt mittels der vorderen Zugeinheit die Orthese um dem Patienten befestigt werden und das erste und das zweite vordere Zugmittel können getrennt voneinander unter Zug gehalten werden. Somit kann eine erste Kraft zur Stabilisierung des Beckens getrennt für das untere und das obere Gurtband eingestellt und auf die individuelle Situation angepasst werden.

Weiter ist vorgesehen, dass die hintere Zugeinheit ein oberes Zugmittel, das auf der Länge des oberen Gurtbands gelegen ist, und ein unteres Zugmittel, das auf der Länge des unteren Gurtbands gelegen ist, aufweist. Das obere und das untere Zugmittel sind durch eine Kraftumlenkung mit einander gekoppelt und können mittels eines gemeinsamen Zugelements, wie etwa einer Zugleine, betätigt werden. Somit sind das untere und das obere Zugmittel voneinander beabstandet, auch beim Einleiten einer Zugkraft auf die hintere Zugeinheit. Durch die Kraftumlenkung wird eine Zugkraft, die durch Ziehen am Zugelement ausgeübt wird, auf die beiden getrennten Zugmittel der hinteren Zugeinheit verteilt. Somit kann beim Anlegen der Orthese in einem zweiten Schritt durch Betätigung des Zugelements die Orthese weiter verengt werden und zwar in einem hinteren Beckenbereich der zur Stabilisierung von Traumapatienten besonders wichtig ist. Zusammengefasst kann bei der Orthese nach der vorliegenden Erfindung eine erste grobe Einstellung durch die vordere Zugeinheit und eine weitere feinere Einstellung durch die hintere Zugeinheit erfolgen.

Vorzugsweise liegt bei dieser Ausführungsform, um eine Zugkraft in die hintere Zugeinheit einzuleiten, ein Zugangriffspunkt des gemeinsamen Zugelements für das obere und untere Zugmittel auf der Länge des unteren Gurtbands. Das Becken ist im Bereich des unteren Gurtbands stabiler gebaut als im Bereich des oberen Gurtbands, so dass ein Angriffspunkt in diesem Bereich beim Festziehen der Orthese weniger unerwünschte Effekte auf das Becken mit sich bringt.

Alternativ zu dieser Ausführungsform ist es auch möglich, dass bei der Zugeinrichtung der Orthese das erste und das zweite vordere Zugmittel gekoppelt sind, so dass das obere und das untere Gurtband gemeinsam festgezogen und befestigt werden können. Eine solche Ausgestaltung beschleunigt zwar den ersten Schritt zum Anlegen der Orthese um den Patienten. Allerdings können das obere und das untere Gurtband dabei nicht unabhängig voneinander angezogen werden. Weiter können alternativ das obere Zugmittel und das untere Zugmittel der hinteren Zugeinheit nicht miteinander gekoppelt sein, so dass sie unabhängig voneinander mit einer unterschiedlichen Zugkraft beaufschlagt werden können. Allerdings verlängert dies die Zeit für das Anlegen der Orthese.

In einer Ausführungsform der Orthese nach der Erfindung ist die hintere Zugeinheit als Seilzug ausgebildet oder, falls die hintere Zugeinheit ein oberes und unteres Zugmittel aufweist, sind oberes und unteres Zugmittel als Seilzug ausgebildet. Eine solche Seilzugeinheit oder ein solches Seilzugmittel ist nach dem Prinzip eines Flaschenzugs aufgebaut. Dabei sind eine erste Seilzugleiste auf einer Seite des mittleren Bandbereichs, der zusammengezogen werden soll, und eine zweite Seilzugleiste auf einer gegenüberliegenden Seite des mittleren Bandbereichs an einem Gurtband oder übergreifend an beiden Gurtbändern angebracht. Die Seilzugleisten weisen mehrere Umlenkpunkte auf, um welche eine Leine, ein Seil oder dergleichen mehrfach zwischen den sich gegenüberliegenden Leisten hin und her geführt ist und in die Zugleine zum Straffen der Seilzugeinheit, bzw. des Seilzugmittels übergeht. Beim Anziehen der Leine wird die erforderliche Kraft zum Zusammenziehen des mittleren Bereichs des Gurtbands über die mehrfache Umlenkung der Leine auf die Gurtbände verteilt, so dass eine geringe Zugkraft zum Straffen der Gurtbände erforderlich ist.

Nach einer bevorzugten Variante weist die Orthese ein oberes Seilzugmittel am oberen Gurtband und ein unteres Seilzugmittel am unteren Gurtband auf, die jeweils eine Zugleine zur Betätigung der Seilzugmittel haben. Bevorzugt werden die Zugleinen miteinander gekoppelt und bilden ein gemeinsames Zugelement zur Betätigung der hinteren Zugeinheit, bei der beide Seilzugmittel gleichzeitig angezogen werden können. Hierfür kann z. B. die obere Zugleine des oberen Seilzugmittels über eine Kraftumlenkung in Richtung der Zugrichtung der unteren Zugleine des unteren Seilzugmittels umgelenkt und mit der unteren Zugleine verbunden werden. Dadurch bilden die obere und die untere Zugleine eine gemeinsame Zugleine für die hintere Zugeinheit. Dadurch wird sichergestellt, dass das obere und das untere Gurtband möglichst parallel gestrafft werden, obgleich nur ein Handgriff zum Anziehen der hinteren Zugeinheit erforderlich ist.

Ein Seilzugmittel für eine Orthese nach der Erfindung weist vorteilhaft eine Kraftübertragung von wenigstens 1:4 und höchstens 1:10 aufweist. Bei einer

Kraftübertragung von 1:4 reduziert sich die zur Verengung der Gurteinheit erforderliche Kraft soweit, dass eine Rettungskraft beim Anlegen der Orthese ohne grosse körperliche Anstrengung die gewünschte Stabilisierung des Beckens herstellen kann. Bei einem Verhältnis der Kraftübertragung von über 1:10 wird jedoch ein an der Zugleine zu ziehender Weg sehr lang, wodurch das Festziehen der Orthese umständlich und langwierig wird.

Nach einer weiteren Ausführungsform der Orthese nach der Erfindung weist eine Zugleine zur Betätigung der hinteren Zugeinheit einen elastischen Bereich auf, der in Zugrichtung dehnbar ist, und die Zugleine weist einen Indikator zur Anzeige der aufgebrachten Zugkraft in Abhängigkeit von der Dehnung des elastischen Bereichs auf. Ein solcher Zugkraftindikator kann bei einem oben erwähnten Seilzug als Zugeinheit oder auch bei anders ausgebildeten Zugeinheiten, wie etwa einer einfachen Zugumlenkung, vorteilhaft zum Einsatz kommen. Beim Ziehen an der Zugleine wird zunächst die Gurteinheit gestrafft bis einem weiteren Ziehen eine Gegenkraft entgegen gesetzt wird. Das weitere Ziehen greift dann am elastischen Bereich der Zugleine an, wodurch dieser gedehnt wird. Die Längenveränderung des elastischen Bereichs kann somit als Mass für eine aufgewendete Zugkraft verwendet werden. Vorzugsweise verläuft zumindest ein Teil des elastischen Bereichs unter einer Abdeckung, so dass die Längenveränderung relativ zur Abdeckung festgestellt werden kann. Besonders bevorzugt ist der elastische Bereich von der Abdeckung überdeckt und tritt bei Überschreiten einer vorbestimmten maximal aufzubringenden Kraft aus der Abdeckung hervor, womit angezeigt wird, dass diese Kraft überschritten wird. Andererseits kann mittels dem Zugkraftindikator sichergestellt werden, dass eine ausreichende Kraft zur Kompression und damit zur Stabilisierung des gesamten Beckens aufgewendet wird.

Bei einer vorteilhaften Ausführungsform der Orthese nach der Erfindung sind im mittleren Bereich der Gurtbänder, d. h. im Bereich der hinteren Zugeinheit, zwei Stützplatten zum Abstützen des hinteren Beckenbereichs vorgesehen. Die Stützplatten erstrecken sich über das obere und das untere Gurtband und sind zu einander entlang der Gurtbänder beabstandet angeordnet. Die Stützplatten sind mittels der hinteren Zugeinheit miteinander verbunden und auf einander zu beweglich. Die Stützplatten gewährleisten den Abstand des oberen und unteren Gurtbands und verteilen die Kompression der Gurteinheit beim Straffen der Bänder auf den hinteren Beckenbereich. Ferner unterstützen sie eine gleichmässige Übertragung des Zugs eines gemeinsamen Zugelements auf ein oberes und ein unteres Zugmittel der hinteren Zugeinheit, wobei sich die Stützplatten gleichmässig aufeinander zu bewegen und den hinteren Beckenbereich verengen und stabilisieren. Mit anderen Worten wirken die Stützplatten als Schubelemente, welche den linken und den rechten Teil des hinteren Beckenbereichs aufeinander zu schieben, dabei jedoch sicherstellen, dass die Gurtbänder im gewünschten Abstand zueinander und somit an der erforderlichen Position am Becken bleiben.

Vorzugweise sind die Stützplatten spiegelsymmetrisch zu einer Achse der Gurteinheit ausgebildet, wobei sie in einem unteren Bereich am unteren Gurtband breiter ausgelegt sind als in einem oberen Bereich am oberen Gurtband. Dadurch bleiben seitlich der Stützplatten Bereiche des Beckens frei, die zur Untersuchung und Behandlung des Patienten relevant sind. Auch bleibt der Bereich zwischen den Stützplatten frei, obgleich er sich beim Straffen der Gurteinheit verkleinert, so dass auch der hintere Beckenbereich in Verlängerung der Wirbelsäule zugänglich bleibt.

Bei einer Variante der Orthese sind das obere und das untere Gurtband im Bereich zwischen den Stützplatten unterbrochen und durch ein oberes Seilzugmittel und ein unteres Seilzugmittel miteinander verbunden. Diese Unterbrechung der Gurtbänder liegt somit im mittleren Bereich der Gurtbänder, der für die hintere Zugeinheit vorgesehen ist. Beispielsweise enden die Gurtbänder an den Seilzugleisten der Seilzugmittel. Da das Seil oder die Leine der Seilzugmittel im Vergleich zum Gurtband deutlich schmaler ist, verbleibt dadurch ein grösserer freier Raum als bei einem durchgängigen Gurtband.

Durch die Ausgestaltung der Orthese mit einem oberen und einem unteren Gurtband sowie Stützplatten zwischen den Gurtbändern in einem hinteren Beckenbereich verbleibt zumindest im vorderen und seitlichen Beckenbereich ein frei zugänglicher Freiraum. Das Becken bleibt somit für eine operative Frakturfixierung vorne und seitlich zugänglich, ohne dass die Orthese entfernt muss. Auch im hinteren Beckenbereich verbleiben im Vergleich zu Beckenorthesen nach dem Stand der Technik wichtige Bereich frei und behindern die weitere Versorgung des Patienten nicht, wobei gleichzeitig eine optimale Stabilisierung des Beckens besteht.

Die Orthese nach der Erfindung ist aus textilen Materialien gefertigt und weist keinerlei Metallelemente auf. Die Orthese kann deshalb bei Aufnahmen mittels Magnetresonanztomographie am Patienten verbleiben und verursacht keine Überlagerungen bei Röntgenaufnahmen.

Die Erfindung wurde an Hand mehrerer Ausführungsformen dargestellt. Die einzelnen technischen Merkmale einer Ausführungsform können durchaus auch in Kombination mit einer anderen Ausführungsform mit den dargelegten Vorteilen verwendet werden. Die Beschreibung der erfindungsgemässen technischen Merkmale ist daher nicht auf die jeweilige Ausführungsform beschränkt.

Eine vorteilhafte Ausführungsform der Erfindung wird im Folgenden anhand der Zeichnungen dargestellt, die lediglich zur Erläuterung dienen und nicht einschränkend auszulegen sind. Aus den Zeichnungen offenbar werdende Merkmale der Erfindung sollen einzeln und in jeder Kombination als zur Offenbarung der Erfindung gehörend betrachtet werden. In den Zeichnungen zeigen:
- Fig. 1: eine schematische Aufsicht auf eine Ausführungsform einer Orthese zur Beckenstabilisation nach der Erfindung in einem geöffneten Zustand,
- Fig. 2: eine schematische Detailansicht einer hinteren Zugeinheit der Ausführungsform aus Figur 1 in einem aufgeweiteten Zustand,
- Fig. 3a: die schematische Aufsicht auf die Orthese aus Figur 1 im aufgeweiteten Zustand und
- Fig. 3b: die schematische Aufsicht auf die Orthese aus Figur 1 und 3a in einem gestrafften Zustand.

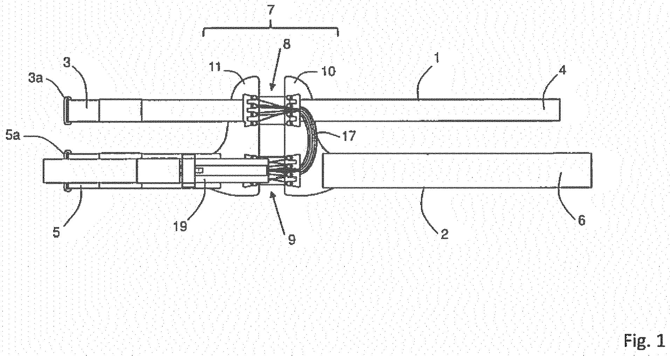

Als Bezugssystem zur Beschreibung einer Ausführungsform der Orthese zur Beckenstabilisation nach der Erfindung mit Hilfe der Figuren 1, 2, 3a und 3b soll die Anordnung der Orthese an einem Patienten verwendet werden. Wie vorher bereits erläutert, soll ein oberer Bereich dem Kopf und ein untere Bereich den Beinen näher sein. Somit liegt z. B. das Kreuzbein oberhalb des Sitzbeins. Ferner sollen ein vorderer Bereich an einer Vorderseite des Patienten und ein hinterer Bereich an einer Hinterseite des Patienten liegen. Demnach liegen z. B. das Kreuzbein hinten und das Schambein vorne.

In Figur 1 ist eine schematische Darstellung einer Ausführungsform einer Orthese zur Beckenstabilisation nach der Erfindung in einem geöffneten, langestreckten Zustand gezeigt, um die einzelnen Elemente der Orthese leichter erläutern zu können. Die Orthese umfasst eine Gurteinheit mit einem oberen Gurtband 1 und einem unteren Gurtband 2. Das obere Gurtband 1 und das untere Gurtband 2 verlaufen im Wesentlichen parallel und beabstandet zu einander. Das untere Gurtband 2, das den unteren Beckenbereich umgibt, ist breiter als das obere Gurtband 1, welches das Becken im Bereich des Kreuzbeins umgibt. Eine vordere Zugeinheit wird von einem ersten vorderen Zugmittel und einem zweiten vorderen Zugmittel gebildet. Das erste vordere Zugmittel umfasst dabei ein Ösenelement 3a, wie etwa eine Schnalle, das an einem Endbereich 3 des oberen Gurtbands 1 befestigt ist, und den gegenüberliegenden Endbereich 4 des oberen Gurtbands 1, der durch das Ösenelement 3a hindurch gezogen werden kann, um das obere Gurtband 1 zu schliessen. Analog umfasst das zweite vordere Zugmittel ein weiteres Ösenelement 5a das an einem Endbereich 5 des unteren Gurtbands 2 befestigt ist, und den gegenüberliegenden Endbereich 6 des unteren Gurtbands 2, der durch das weitere Ösenelement 5a hindurch gezogen werden kann, um das untere Gurtband 2 zu schliessen. Beim Anlegen der Orthese an einem Patienten werden z. B. die Endbereiche 4 und 6 des oberen und unteren Gurtbands um das Becken gelegt und durch ihr zugehöriges Ösenelement 3a und 5a gezogen, so dass die Gurtbänder eine Schlaufe um das Becken bilden, wobei die Endbereiche der Gurtbänder 1, 2 an einem vorderen Beckenbereich liegen. Dabei kommt möglichst das obere Gurtband 1 auf Höhe des Kreuzbeins und das untere Gurtband 2 auf Höhe der Oberschenkelköpfe und der Schambeine zu liegen. Die Endbereiche 3 und 4 des oberen Gurtbands 1 bilden somit gemeinsam mit dem Ösenelement 3a das erste vordere Zugmittel. Analog bilden die Endbereiche 5 und 6 des untern Gurtbands 2 gemeinsam mit dem Ösenelement 5a das zweite vordere Zugmittel.

Die vordere Zugeinheit umfasst weiter eine Befestigungseinrichtung, um das obere und das untere Gurtband 1, 2 unter Zug um das Becken zu befestigen. Hierfür kann z. B. ein Klettverschluss vorgesehen sein, indem die Endbereiche 4 und 6 am Ende der Gurtbänder eine Fläche mit Widerhaken und daran anschliessend eine Fläche mit Ösen aufweisen. Sobald ein Endbereich 4, 6 mit dem Klettverschluss durch das zugehörige Ösenelement 3a, 5a gezogen ist, kann er unter Zug umgefaltet werden, so dass die Widerhakenfläche auf der Schlaufenfläche fixiert werden kann.

Zur Ausbildung der vorderen Zugmittel könnte anstelle des Ösenelements und eines Klettverschlusses z. B. auch ein Schnappverschluss oder dergleichen verwendet werden, bei welchem ein erstes Schnappelement am Endbereich 3 bzw. 5 der Gurtbänder und ein zweites Schnappelement am gegenüberliegenden Endbereich 4 bzw. 6 angebracht ist, wobei die Schnappelemente zur Ausbildung eines Verschlusses lösbar miteinander verschnappen können. Das Schnappelement am Endbereich 4 bzw. 6 weist eine Rückrutschsicherung auf, so dass dieser Endbereich durch das Schnappelement in einer Richtung durchgezogen und festgehalten werden kann, wobei in Gegenrichtung aber ein Zurückrutschen des Endbereichs blockiert.

In einem mittleren Bereich 7 der Gurtbänder 1 und 2 ist eine hintere Zugeinheit zum Straffen der Gurteinheit im hinteren Beckenbereich vorgesehen. In einem am Patienten angelegten Zustand der Orthese liegt die hintere Zugeinheit an der hinteren Seite des Beckens. Die hintere Zugeinheit umfasst ein oberes Zugmittel in Form eines oberen Seilzugmittels 8 am oberen Gurtband 1 und ein unteres Zugmittel in Form eines unteren Seilzugmittels 9 am unteren Gurtband 2. Weiter sind in dem mittleren Bereich 7 zwei spiegelsymmetrisch geformte Stützplatten 10 und 11 angebracht. In einem am Patienten angelegten Zustand der Orthese liegen die Stützplatte 10 auf der rechten Seite des Kreuzbeins und der Lendenwirbel und die Stützpatte 11 auf der linken Seite. Beim Straffen des mittleren Bereichs 7 durch die Seilzugmittel 8 und 9 werden die Stützplatten 10 und 11 aufeinander zu bewegt, so dass der hintere Beckenbereich komprimiert und stabilisiert wird.

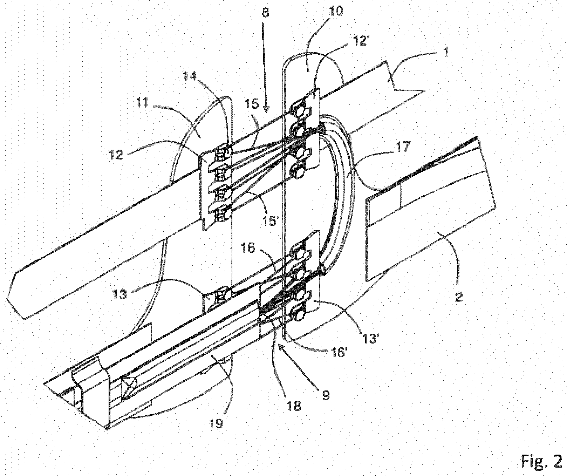

In Figur 2 ist der mittlere Bereich 7 mit der hinteren Zugeinheit und den Stützplatten 10 und 11 im Detail gezeigt. Daraus wird ersichtlich, dass die Gurtbänder 1 und 2 im Bereich zwischen den Stützplatten 10 und 11 unterbrochen und durch die Seilzugmittel 8 und 9 verbunden sind. Das Seilzugmittel 8 weist eine erste Seilzugleiste 12 auf der linken Stützplatte 11 und eine zweite Seilzugleiste 12' auf der rechten Stützplatte 10 auf, die beide quer zur Längsrichtung des oberen Gurtbands 1 und entlang dessen Länge auf den Stützplatten befestigt sind. Entsprechend weist das Seilzugmittel 9 zwei Seilzugleisten 13 und 13' auf, die beide quer zur Längsrichtung des unteren Gurtbands 2 und entlang dessen Länge auf den Stützplatten befestigt sind. Die Seilzugleisten 12, 12', 13 und 13' weisen eine Reihe von mehreren Umlenkpunkten 14 auf, die für die Umlenkung eines oder mehrerer Zugseile vorgesehen sind. Bei der dargestellten Ausführungsform der Orthese sind vier Umlenkpunkte 14 vorgesehen. Es könnten aber auch mehr oder weniger Umlenkpunkte verwendet werden, solange ein Seilzug nach der Art eines Flaschenzugs für die hintere Zugeinheit realisiert werden kann.

Bei der gezeigten Variante werden bei einem Seilzugmittel zwei Zugleinen verwendet, um zwei nebeneinander liegende Seilzüge zwischen den Seilzugleisten herzustellen. Beim oberen Seilzugmittel 8 werden Zugleinen 15 und 15' zwischen den mittleren Umlenkpunkten 14 auf der Seilzugleiste 12' an das Seilzugmittel 8 herangeführt. Die beiden Zugleinen 15 und 15' werden abwechselnd zwischen den Umlenkpunkten 14 der linken Seilzugleiste 12 und der rechten Seilzugleiste 12' hin und her geführt bis ein Zugkraftverhältnis von 1:4 vorliegt. Dabei verläuft die Zugleine 15 um die oberen zwei Umlenkpunkte 14 der Seilzugleisten 12 und 12' und Zugleine 15' um die unteren zwei Umlenkpunkte 14 der Seilzugleisten 12 und 12'. Die Zugleinen 15 und 15' werden zwischen den mittleren Umlenkpunkten 14 der Seilzugleiste 12' zurückgeführt. Die Krafteinwirkung bei einem Anziehen der Zugleinen 15 und 15' wird durch deren Führung zwischen den Umlenkpunkten 14 über die gesamte Breite des oberen Gurtbands 1 verteilt. In vergleichbarer Weise werden für das untere Seilzugmittel 9 zwei Zugleinen 16 und 16' abwechselnd zwischen den Umlenkpunkten 14 der linken Seilzugleiste 13 und der rechten Seilzugleiste 13' hin und her geführt, so dass die Krafteinwirkung bei Zug an den Zugleinen 16 und 16' über die Breite des unteren Gurtbands 2 verteilt wird. Die Umlenkpunkte 14 können als einfache Rundungen an Noppen oder der gleichen oder als Rollen ausgebildet sein. Die Zugleinen überbrücken den Zwischenraum zwischen den Stützplatten auf der Höhe der Gurtbänder und bilden somit einen Teil der Gurtbänder.

Die Zugleinen 15 und 15' treten zumindest annähernd mittig zur Breite des oberen Gurtbands 1 in das Seilzugmittel 8 ein und wieder aus. Über eine Führung 17 werden die Enden der Zugleinen 15 und 15' vom oberen Gurtband 1 zum unteren Gurtband 2 umgelenkt und zum Zugangriffspunkt 18 der Zugleinen 16 und 16' des Seilzugmittels 9 geführt. Die Führung kann z. B. von einem gebogenen Kanal gebildet werden. Der Zugangriffspunkt 18 der Zugleinen 16 und 16' liegt zumindest annähernd mittig zur Breite des Gurtbandes 2. An diesem Zugangriffspunkt 18 laufen alle Zugleinen zusammen und können gleichzeitig durch Zug in dieselbe Richtung angezogen werden. Ab dem Zugangriffspunkt 18 bilden die Zugleinen ein gemeinsames Zugelement, z. B. in Form eines Zugbandes 19, mit dem das obere und das untere Seilzugmittel 8 und 9 gleichzeitig betätigt werden können. Eine auf das Zugband 19 einwirkende Zugkraft wirkt dabei in gleicher Zugrichtung auf die Zugleinen 16 und 16' des unteren Seilzugmittels 9. Die Zugkraft wird jedoch über die Führung 17 um 180° zum oberen Seilzugmittel 8 umgelenkt, so dass die Zugrichtung auf die Zugleinen 15 und 15' des oberen Seilzugmittels 8 in die entgegengesetzte Richtung wirkt. Zusammengefasst ermöglicht es die Kraftumlenkung, dass das untere Gurtband 2 durch Zug am unteren Zugmittel in einer Zugrichtung und das obere Gurtband 1 durch Zug am oberen Zugmittel in entgegen gesetzter Zugrichtung gestrafft wird. Die Zugkräfte am unteren und oberen Gurtband wirken somit gegenläufig und die Gefahr den Patienten beim Straffen der hinteren Zugeinheit zu drehen, wie es bei einem Zug in gleicher Zugrichtung der Fall sein könnte, wird vermindert.

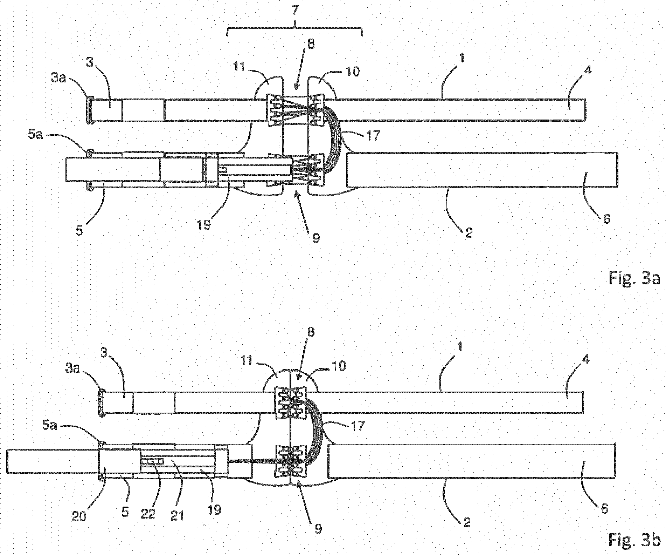

Aus Figur 2 ist auch die Ausgestaltung der Stützplatten 10 und 11 ersichtlich. Die Stützplatten 10 und 11 erstrecken sich über das obere und das untere Gurtband 1 und 2, so dass die Gurtbänder 1 und 2 zueinander beabstandet gehalten werden. Der Abstand beträgt mindestens 5 cm, vorzugsweise liegt der Abstand um 10 cm. Die Stützplatten 10 und 11 sind in Figur 2 und 3a in einem aufgeweiteten Zustand der hinteren Zugeinheit gezeigt, in dem sie weit voneinander entfernt angeordnet sind. In Figur 3b ist die hintere Zugeinheit in einem angezogenen Zustand gezeigt, in dem die Stützplatten 10 und 11 weniger weit voneinander entfernt angeordnet sind; in der dargestellten Position liegen die Stützplatten für leichtere Verständlichkeit beinahe aneinander an. Wie oben geschildert sind die Stützplatten 10 und 11 mittels der hinteren Zugeinheit verbunden und auf einander zu beweglich, wobei mittels des gemeinsamen Zugbands beide Seilzugmittel 8 und 9 gleichmässig angezogen werden können und sich die Stützplatten 10 und 11 zumindest annähernd parallel aufeinander zu bewegen. Die Stützplatten 10 und 11 stützen zum einen die Gurtbänder relativ zueinander und zum anderen dienen sie beim Straffen der hinteren Zugeinheit zur gleichmässigen Übertragung der Kompressionskraft auf den hinteren Beckenbereich.

Die Stützplatten 10 und 11 sind spiegelsymmetrisch zu einer Längsachse durch die Gurteinheit ausgebildet. In einem unteren Bereich am unteren Gurtband 1 sind die Stützplatten 10 und 11 breiter ausgelegt als in einem oberen Bereich am oberen Gurtband 2. Insgesamt sind das obere Gurtband 1 und das untere Gurtband 2 und die Stützplatten 10 und 11 derart angeordnet, dass im vorderen und seitlichen Beckenbereich ein frei zugänglicher Freiraum zwischen den Gurtbänder ausgebildet ist und eine möglichst geringe Abdeckung im hinteren Beckenbereich durch die Stützplatten erfolgt. Auch wird der freie Raum zwischen den Stützplatten 10 und 11 erhöht, indem die Gurtbänder dort unterbrochen und durch die Zugleinen 15, 15' und 16, 16' ersetzt sind. Der Freiraum ist zur Versorgung des Patienten wichtig, wie eingangs erläutert.

In Figur 3a ist die hintere Zugeinheit der Orthese in einem weit geöffneten Zustand gezeigt. Die Figur 3a entspricht der Figur 1. Das Zugband 19 zur Betätigung der Seilzugmittel 8 und 9 ist nicht angezogen und liegt in Figur 3a weiter rechts. Demgegenüber ist die hintere Zugeinheit der Orthese in Figur 3b in einem annähernd geschlossenen Zustand gezeigt. Das Zugband 19 ist angezogen und die Seilzugmittel 8 und 9 sind gestrafft, wobei das Zugband 19 in Figur 3b weiter links liegt. Grundsätzlich ist ein vollständiges Schliessen der hinteren Zugeinheit, so dass die Stützplatten 10 und 11 aneinander anstossen, zu vermeiden, da dann eine weitere Feineinstellung der Kompressionskraft der Orthese nicht weiter möglich ist. Obgleich in Figur 3b nicht gezeigt, sind natürlich in einem Zustand, in dem die Orthese einen Patienten umschliesst und zur Stabilisierung gestrafft ist, das obere Gurtband 1 und das untere Gurtband 2 mittels der Ösenelemente 3a und 5a und der Befestigungseinrichtung geschlossen.

Das Zugband 19 weist einen elastischen Bereich 21 auf, der in Zugrichtung dehnbar ist und in entspanntem Zustand unter einer Abdeckung 20 liegt. Die Abdeckung 20 ist am Zugband 19 befestigt. Als elastischer Bereich kann z. B. ein Gummiband in das Zugband eingesetzt werden. Weiter weist das Zugband 19 einen Indikator 22 zur Anzeige einer aufgebrachten Zugkraft in Abhängigkeit von der Dehnung des elastischen Bereichs 21 auf. Der Indikator 22 kann beispielsweise durch den elastischen Bereich selbst oder einer Markierung, z. B. in Form eines Farbstreifens, auf dem elastischen Bereich gebildet werden. Wie in Figur 3a ersichtlich, liegt der elastische Bereich 21 unter der Abdeckung 20, wenn das Zugband 19 nicht angezogen ist. Auch der Indikator 22 ist dann von der Abdeckung 20 verdeckt. Wird das Zugband 19 angezogen und überschreitet die Zugkraft eine vordefinierte Kraft, wird der elastischen Bereich 21 gedehnt und tritt aus der Abdeckung hervor, so dass er sichtbar wird. Einer Hilfsperson, die einem Patienten die Orthese nach der Erfindung anlegt, wird damit angezeigt, dass die vordefinierte Zugkraft erreicht ist und bei weiterem Ziehen überschritten wird. Damit kann zu starkes Straffes der Orthese vermieden werden.

Um das Zugband 19 in einem angezogen Zustand wie bei Figur 3b zu befestigen, ist eine Befestigungseinheit vorgesehen, die das Zugband 19 am unteren Gurtband 2 befestigt. Als Befestigungseinheit kann wieder ein Klettverschluss verwendet werden, wobei z. B. ein eine Widerhakenfläche am Zugband 19 mit einer Schlaufenfläche am unteren Gurtband zusammenwirkt. Alternativ könnte auch eine Haken- oder Knopfverbindung als Befestigungseinheit zwischen Zugband 19 und unterem Gurtband 2 dienen.

**Bezugszeichen**

| | | | |
|---|---|---|---|
| 1 | oberes Gurtband | 11 | Stützplatte |
| 2 | unteres Gurtband | 12, 12' | Seilzugleisten |
| 3 | Endbereich | 13, 13' | Seilzugleisten |
| 3a | Ösenelement | 14 | Umlenkpunkt |
| 4 | Endbereich | 15, 15' | Zugleinen oben |
| 5 | Endbereich | 16, 16' | Zugleinen unten |
| 5a | Ösenelement | 17 | Führung |
| 6 | Endbereich | 18 | Zugangriffspunkt |
| 7 | mittlerer Bereich | 19 | Zugband |
| 8 | oberes Seilzugmittel | 20 | Abdeckung |
| 9 | unteres Seilzugmittel | 21 | elastischer Bereich |
| 10 | Stützplatte | 22 | Indikator |

## Patentansprüche

1. Orthese zur Beckenstabilisation, die eine Gurteinheit, die zum Umschliessen des Beckens vorgesehen ist, und eine Zugeinrichtung, die zum Straffen der Gurteinheit vorgesehen ist, aufweist, wobei
- die Gurteinheit ein oberes Gurtband (1), das einen oberen Beckenbereich umschliesst, und ein unteres Gurtband (2), das einen unteren Beckenbereich umschliesst, umfasst, und
- die Zugeinrichtung eine vordere Zugeinheit mit einer Befestigungseinrichtung umfasst, wobei die vordere Zugeinheit an einem vorderen Beckenbereich der Gurteinheit angeordnet ist und das obere und das untere Gurtband in einem vorderen Beckenbereich durch Zug strafft, und wobei die Befestigungseinrichtung die gegenüberliegende Enden (3, 4; 5, 6) des oberen und des unteren Gurtbands im vorderen Beckenbereich miteinander verbindet und unter Zug befestigt,
**dadurch gekennzeichnet, dass** die Zugeinrichtung weiter aufweist:
- eine hintere Zugeinheit (8, 9), die in einem mittleren Bereich (7) der Bänder am oberen Gurtband (1) und am unteren Gurtband (2) angeordnet und zum Straffen eines hinteren Beckenbereichs der Gurteinheit vorgesehen ist, und
- eine Befestigungseinheit, welche die hintere Zugeinheit unter Zug befestigt.

2. Orthese nach Anspruch 1, **dadurch gekennzeichnet, dass** die hintere Zugeinheit (8, 9) übergreifend am oberen Gurtband (1) und unteren Gurtband (2) angeordnet ist, so dass die hintere Zugeinheit beim Straffen des mittleren Bandbereichs (7) gleichzeitig auf das obere und das untere Gurtband wirkt.

3. Orthese nach einem der vorhergehenden Ansprüche, **dadurch gekennzeichnet, dass** die vordere Zugeinheit ein erstes vorderes Zugmittel (3a, 4) am oberen Gurtband (1) für einen vorderen Beckenbereich und ein zweites vorderes Zugmittel (5a, 6) am unteren Gurtband (2) ebenfalls für einen vorderen Beckenbereich aufweist.

4. Orthese nach einem der vorhergehenden Ansprüche, **dadurch gekennzeichnet, dass** die hintere Zugeinheit ein oberes Zugmittel, das auf der Länge des oberen Gurtbands (1) gelegen ist, und ein unteres Zugmittel, das auf der Länge des unteren Gurtbands (2) gelegen ist, aufweist, wobei das obere und das untere Zugmittel durch eine Kraftumlenkung (17) mit einander gekoppelt sind und mittels eines gemeinsamen Zugelements (19) betätigbar sind.

5. Orthese nach Anspruch 4, **dadurch gekennzeichnet, dass** ein Zugangriffspunkt (18) der gemeinsamen Zugelements (19), um eine Zugkraft in die hintere Zugeinheit (8, 9) einzuleiten, auf der Länge des unteren Gurtbands (2) vorgesehen ist.

6. Orthese nach einem der vorhergehenden Ansprüche, **dadurch gekennzeichnet, dass** die hintere Zugeinheit oder das obere und unter Zugmittel als Seilzug ausgebildet ist bzw. sind.

7. Orthese nach Anspruch 6, **dadurch gekennzeichnet, dass** ein oberes Seilzugmittel (8) und ein unteres Seilzugmittel (9) der hinteren Zugeinheit über wenigstens ein gemeinsames Zugelement (19) miteinander gekoppelt sind.

8. Orthese nach Anspruch 6 oder 7, **dadurch gekennzeichnet, dass** eine obere Zugleine (15, 15') für das obere Seilzugmittel (8) über eine Kraftumlenkung (17) zu einer unteren Zugleine (16, 16') für das unter Seilzugmittel (8) geführt ist und die obere und untere Zugleine in das gemeinsame Zugelement (19) übergehen.

9. Orthese nach einem der Ansprüche 6 bis 8, **dadurch gekennzeichnet, dass** ein Seilzugmittel (8, 9) eine Kraftübertragung von wenigstens 1:4 und höchstens 1:10 aufweist.

10. Orthese nach einem der vorhergehenden Ansprüche, **dadurch gekennzeichnet, dass** ein Zugelement (19) zur Betätigung der hinteren Zugeinheit (8, 9) einen elastischen Bereich (21) aufweist, der in Zugrichtung dehnbar ist, und das Zugelement (19) einen Indikator (22) zur Anzeige der aufgebrachten Zugkraft in Abhängigkeit von der Dehnung des elastischen Bereichs (21) aufweist.

11. Orthese nach Anspruch 10, **dadurch gekennzeichnet, dass** zumindest ein Bereich des elastischen Bereichs (21) des Zugelements (19) unter einer Abdeckung (20) verläuft.

12. Orthese nach einem der vorhergehenden Ansprüche, **dadurch gekennzeichnet, dass** im mittleren Bereich (7) der Gurtbänder zwei Stützplatten (10, 11) zum Abstützen des hinteren Beckenbereichs vorgesehen sind, die sich über das obere Gurtband (1) und das untere Gurtband (2) erstrecken, zu einander beabstandet angeordnet sind und mittels der hinteren Zugeinheit (8, 9) verbunden und auf einander zu beweglich sind.

13. Orthese nach Anspruch 12, **dadurch gekennzeichnet, dass** die Stützplatten (10, 11) spiegelsymmetrisch zu einer Achse der Gurteinheit ausgebildet sind, wobei die Stützplatten (10, 11) in einem unteren Bereich am unteren Gurtband (2) breiter ausgelegt sind als in einem oberen Bereich am oberen Gurtband (1).

14. Orthese nach Anspruch 12 oder 13, **dadurch gekennzeichnet, dass** das obere Gurtband (1) und das untere Gurtband (2) im Bereich zwischen den Stützplatten (10, 11) unterbrochen sind und durch ein oberes Seilzugmittel (15, 15') und ein unteres Seilzugmittel (16, 16') miteinander verbunden sind.

15. Orthese nach einem der vorhergehenden Ansprüche, **dadurch gekennzeichnet, dass** das obere Gurtband (1) und das untere Gurtband (2), und gegebenenfalls die Stützplatten (10, 11), derart angeordnet sind, dass zumindest im vorderen und seitlichen Beckenbereich ein frei zugänglicher Freiraum ausgebildet ist.
